# EUROPEAN PATENT APPLICATION

(11) **EP 1 092 766 A1**
(43) Date of publication of application: **18.04.2001**
(21) Application number: 99926914.5
(22) Date of filing: 02.07.1999
(51) Int. Cl.: C12N 1/20, C12P 17/00, C07C 62/04

(54) **MICROORGANISM BELONGING TO THE GENUS CITROBACTER AND PROCESS FOR PRODUCING SHIKIMIC ACID**

(30) Priority: 02.07.1998 JP 18746498
(71) Applicant: TORAY INDUSTRIES, INC., Tokyo 103-8666 (JP)
(72) Inventor: SHIRAI, Makoto, Anjo-shi Aichi 446-0071 (JP); MIYATA, Reiko, Aichi 474-0082 (JP); SASAKI, Satoshi, Aichi 493-0001 (JP); SAKAMOTO, Kosuke, Nagoya-shi Aichi 458-0832 (JP); YAHANDA, Saburo, Aichi 468-0011 (JP); SHIBAYAMA, Katsuhiro, Nagoya-shi Aichi 467-0065 (JP); YONEHARA, Tetsu, Nagoya-shi Aichi 457-0036 (JP); OGAWA, Kenichi, Inabe-gun Mie 511-0232 (JP)
(74) Representative: Denison, Christopher Marcus
(86) International application number: JP9903595
(87) International publication number: WO0001800

(57) **Abstract**

Shikimic acid is economically produced using a microorganism belonging to the genus Citrobacter and capable of extracellularly secreting shikimic acid.

## Description

### Technical Field

Shikimic acid is an optically active substance which has three asymmetric carbons and is used in, for example, pharmaceutical materials.

### Background Art

Hitherto, as processes for producing shikimic acid, a process in which shikimic acid is extracted from plants, a synthetic process using quinic acid or a saccharide (Chemical Review 65 435(1965)), and a production process in which shikimic acid is produced by fermentation using *Escherichia coli* (Journal Biological Chemistry 181 315(1951)) are known. As conventional processes for isolating and purifying shikimic acid, a process in which an aqueous solution is heated and refluxed, and shikimic acid is purified by a treatment with activated carbon and with an ion exchange resin-acetic acid eluent (Journal of American Chemical Society vol. 121, pp. 1603 (1999)) is known. In addition, a purification process in which shikimic acid is purified on an activated carbon column (Journal of Biological Chemistry 220 477(1956)) is also known.

According to the extraction process from plants, shikimic acid cannot be significantly purified as such plants have a low shikimic acid content. In the synthetic process using quinic acid or a saccharide as a material, the material is expensive and the process requires a long series of synthesis steps. According to the production process by fermentation using *Escherichia coli*, the concentration of accumulated shikimic acid is low. The conventional isolation and purification process requires a large quantity of energy to heat and reflux the aqueous solution, or requires a large quantity of energy to concentrate and recover acetic acid as the process requires the use of a large amount of expensive acetic acid. The purification process using column chromatography on an activated carbon column have much industrial and economical problems, as the used activated carbon cannot be regenerated.

### Disclosure of Invention

To solve the above problems, the present inventors sought microorganisms other than *Escherichia coli*, which microorganisms have the potential to produce shikimic acid, and made wide investigations on microorganisms which extracellularly secrete shikimic acid. As a result, they found that mutants of a microorganism belonging to the genus Citrobacter extracellularly secrete shikimic acid.

Specifically, the present invention provides, as an embodiment, a microorganism belonging to the genus Citrobacter and capable of extracellularly secreting shikimic acid, and a process for producing shikimic acid using the same.

The process for producing shikimic acid using the microorganism herein naturally includes a process in which shikimic acid is extracellularly produced and accumulated by a conventional fermentation technique, that is, a technique in which a carbon source and a nitrogen source, for example, are added and the microorganism produces a product while growing. The invented process also includes a process in which shikimic acid is produced from a carbon source and is extracellularly secreted by the use of cells, cultures, or treated products thereof under conditions where the growth of the microorganism substantially ceases and an enzyme reaction alone proceeds. This process is different from the conventional fermentation process. In this connection, the term "extracellularly secretes shikimic acid" means that shikimic acid is produced in a medium in the culture solution, that is, shikimic acid is produced and accumulated in a supernatant of the culture, from which the microorganism is removed by, for example, centrifugal separation or filtration.

The present inventors made intensive investigations to further improve the production efficiency of shikimic acid, and found that a mutant resistant to pyruvic acid analogues, a mutant requiring succinic acid or glutamic acid for its growth, a mutant resistant to vitamin B1 analogues, or a mutant resistance to a highly concentrated shikimic acid respectively exhibit a greatly improved production efficiency of shikimic acid. Specifically, the invented microorganism should preferably have any of the following properties:
(1) the property of requiring an aromatic amino acid and/or p-aminobenzoic acid for its growth;
(2) the property of being resistant to a pyruvic acid analogue;
(3) the property of requiring succinic acid for its growth;
(4) the property of requiring glutamic acid for its growth;
(5) the property of being resistant to a vitamin B1 analogue; and
(6) the property of being resistant to a highly concentrated shikimic acid.

On the other hand, the present inventors made intensive investigations to improve the production efficiency of shikimic acid in the mutants capable of extracellularly secreting shikimic acid, and found that the addition of a trace amount of a transition metal to a medium increases productivity of shikimic acid. Specifically, according to the present invention, the microorganism is preferably cultured in a medium in the presence of a transition metal to produce and accumulate shikimic acid to thereby isolate and recover shikimic acid.

The present inventors made further intensive investigations to solve the above problems accompanied with purification on processes for purifying shikimic acid in which impurities are separated and removed from a mixture containing shikimic acid, and found that the use of an aqueous basic solution as an eluent permits dehydroxylation of an impurity 3-dehydroshikimic acid to thereby recover a high-purity shikimic acid in a good yield. Specifically, the present invention provides, as another embodiment, a process for producing shikimic acid which includes the step of subjecting a solution containing shikimic acid to a basic condition to thereby purify shikimic acid. The shikimic acid is further preferably purified by subjecting a solution containing shikimic acid to a basic condition, which shikimic acid is produced by the aforementioned process for producing shikimic acid using the microorganism.

### Best Mode for Carrying Out the Invention

Microorganisms for use in the present invention may be any microorganism belonging to the genus Citrobacter, but microorganisms which strongly biosynthesize aromatic amino acids are preferred, as shikimic acid is a metabolic intermediate in a biosynthetic system of aromatic amino acids. A typical example of shikimic acid-secreting strains is *Citrobacter freundii* 4AA-12 (FERM BP-6722) strain. This mutant is a shikimic acid-secreting mutant and has been obtained by a mutation treatment process using a OFPR36-158 strain as a parent strain, which OFPR36-158 strain has been obtained from *Citrobacter freundii* IFO 13545 strain by acquiring the property of being resistant to 5-fluorotryptophan and of being resistant to o-fluorophenylalanine to thereby become an aromatic amino acid-producing strain.

To induce such mutants, a parent strain is irradiated with ultraviolet ray or is treated with a mutagenic agent (e.g., N-methyl-N'-nitro-N-nitrosoguanidine or ethylmethanesulfonic acid), followed by a conventional procedure to yield shikimic acid-secreting mutants. For example, the parent strain is subjected to a mutation inducing treatment (a treatment with a mutagen), and is then allowed to grow on an agar medium such as a natural medium on which the microorganism can satisfactorily grow, and the grown colonies are replicated on a minimum medium originally containing four aromatic amino acids (L-tyrosine, L-phenylalanine, and L-tryptophan) and p-aminobenzoic acid and on a minimum medium containing none of these amino acids and vitamin, and colonies which only grow on the minimum medium containing these amino acids and vitamin are harvested.

Pyruvic acid analogues for use in the present invention are structural analogues of pyruvic acid and are compounds which inhibit the growth of microorganisms, and the inhibited growth is recovered by the addition of pyruvic acid to a medium. Any compound having these properties can be used, and examples thereof include bromopyruvic acid, fluoropyruvic acid, and phenylpyruvic acid.

The term "pyruvic acid analogue-resistant mutant" means a mutant which can grow even in such a concentration of the compound as to inhibit the growth of a parent strain of the microorganism.

A typical example of shikimic acid-secreting strains which are resistant to pyruvic acid analogues for use in the present invention is *Citrobacter freundii* BPR-3 (FERM BP-6723) strain. This mutant is a mutant derived from *Citrobacter freundii* 4AA-12 (FERM BP-6722) as a parent strain.

To induce such mutants, a parent strain is irradiated with ultraviolet ray or is treated with a mutagenic agent (e.g., N-methyl-N'-nitro-N-nitrosoguanidine or ethylmethanesulfonic acid), and a microorganism is then harvested, which microorganism has grown on a medium containing a pyruvic acid analogue in such a concentration as to inhibit the growth of the parent stain.

The term "mutant having the property of requiring succinic acid and/or glutamic acid for its growth" means a mutant which cannot grow when the medium does not contain the compound, and also includes a "leaky" strain which can slightly grow, as well as a mutant which can grow with the addition of a natural component containing succinic acid or glutamic acid.

A typical example of such shikimic acid-secreting strains for use in the present invention is *Citrobacter freundii* SUC-87 (FERM BP-6724) strain. This mutant is a mutant derived from *Citrobacter freundii* strain 4AA-12 (FERM BP-6722) as a parent strain.

To induce such mutants, a parent strain is irradiated with ultraviolet ray or is treated with a mutagenic agent (e.g., N-methyl-N'-nitro-N-nitrosoguanidine or ethylmethanesulfonic acid), and a mutant is harvested, which mutant cannot grow on a medium containing neither succinic acid nor glutamic acid and can grow on a medium containing succinic acid or glutamic acid.

Vitamin B1 analogues for use in the present invention are structural analogues of Vitamin B1 and are compounds which inhibit the growth of microorganisms, and the inhibited growth is recovered by the addition of Vitamin B1 to a medium. Any compound having these properties can be used, and examples thereof include oxythiamine and thiothiamine. The term "vitamin B1 analogue-resistant mutant" means a mutant which can grow even in such a concentration of the compound as to inhibit the growth of a parent strain of the microorganism.

A typical example of such shikimic acid-secreting strains which are resistant to vitamin B1 analogues for use in the present invention is *Citrobacter freundii* OT-H24 (FERM BP-6711) strain. This mutant is a mutant derived from *Citrobacter freundii* 4AA-12 (FERM BP-6722) as a parent strain. To induce such mutants, a parent strain is irradiated with ultraviolet ray or is treated with a mutagenic agent (e.g., N-methyl-N'-nitro-N-nitrosoguanidine or ethylmethanesulfonic acid), and a microorganism is then harvested, which microorganism has grown on a medium containing a vitamin B1 analogue in such a concentration as to inhibit the growth of the parent strain.

The term "strain resistant to a highly concentrated shikimic acid" for use in the present invention means a mutant which can grow on a medium containing a highly concentrated shikimic acid, but whose parent strain cannot grow on a medium containing the highly concentrated shikimic acid.

A typical example of such shikimic acid-secreting strains which are resistant to a highly concentrated shikimic acid for use in the present invention is *Citrobacter freundii* HSK-10 (FERM BP-6712) strain. This mutant is a mutant derived from *Citrobacter freundii* 4AA-12 (FERM BP-6722) as a parent strain. To induce such mutants, a parent strain is irradiated with ultraviolet ray or is treated with a mutagenic agent (e.g., N-methyl-N'-nitro-N-nitrosognanidine or ethylmethanesulfonic acid), and a microorganism is then harvested, which microorganism has satisfactorily grown on a medium containing shikimic acid in such a concentration as to inhibit or retard the growth of the parent strain.

A culturing process in the invented fermentation process will now be described. As media for the production of shikimic acid, conventional media each containing a carbon source, a nitrogen source, inorganic ions, and additional organic minor components according to necessity can be employed.

Preferred media each appropriately comprise 1 to 15% of carbon sources such as glucose, fructose, hydrolysates of starch and cellulose, molasses and other saccharides, fumaric acid, citric acid, succinic acid, and other organic acids, methanol, ethanol, glycerol, and other alcohols; 0.1 to 4.0% of nitrogen sources such as ammonium acetate and other organic ammonium salts, ammonium sulfate, ammonium chloride, ammonium phosphate, ammonium nitrate, and other inorganic ammonium salts, ammonia gas, aqueous ammonia, and urea; and as organic minor components, 0.000001% to 0.1% of biotin and other required substances, and 0 to 5% of corn steep liquor, peptone, and yeast extract, according to necessity. The media should preferably further comprise minor components such as potassium phosphate, magnesium sulfate, calcium chloride, sodium chloride, zinc sulfate, copper sulfate, and ferrous sulfate. The media further preferably comprise defoaming agents and others to stabilize culture conditions.

Culturing is generally performed under an aerobic condition. During culturing, the pH value is controlled between 3 and 8, and the culture temperature is controlled between 20°C and 40°C culturing is carried out for 24 to 144 hours with shaking or with aeration and agitation.

In addition, the present invention can efficiently produce shikimic acid by culturing a microorganism in a medium for shikimic acid fermentation, which microorganism is capable of extracellularly secreting shikimic acid in the presence of a trace amount of a transition metal, to thereby produce and accumulate shikimic acid, and isolating and recovering shikimic acid. The transition metal is preferably at least one selected from transition metals of Group VIIB, Group VIII, Group IB, and Group IIB. Transition metals of Group VIIB include manganese, technetium, and rhenium. Transition metals of Group VIII include iron, cobalt, nickel, ruthenium, rhodium, palladium, osmium, iridium, and platinum. Transition metals of Group IB include copper, silver, and gold; and transition metals of Group IIB include zinc, cadmium, and mercury. Among them, iron, manganese, copper, and zinc are especially preferred.

The transition metal is preferably added to the medium for shikimic acid fermentation in the form of a water-soluble salt. While depending on the type of the metal, the amount of the transition metal is preferably 0.1 mg/L to 500 mg/L for zinc.

The transition metal may be added in early stages of or during culturing, but it is preferably added in early stages of culturing. The term "extracellularly secretes shikimic acid" means the microorganism produces shikimic acid in a medium of the culture, i.e., the shikimic acid is produced and accumulated in a supernatant of the culture from which the microorganism is removed by centrifugal separation or filtration.

The culturing process in the invented fermentation process will now be described. As media for the production of shikimic acid, conventional media each containing a carbon source, a nitrogen source, inorganic ions, and additional organic minor components according to necessity can be employed.

In the invented process for producing shikimic acid, it is important to purify shikimic acid by subjecting a solution containing shikimic acid to a basic condition. As the basic condition, the pH of the solution is preferably 8 or more, and more preferably 10 or more. A reaction temperature is preferably from room temperature to reflux temperature and is more preferably 40°C or more in order to complete the reaction in a short time. The solution should be preferably subjected to a basic condition by the use of an aqueous solution of a metal hydroxide. Such metal hydroxides include, for example, sodium hydroxide, potassium hydroxide, calcium hydroxide, magnesium hydroxide, aluminium hydroxide, and barium hydroxide. More preferably, the solution is subjected to a basic condition by bringing the solution into contact with a basic anion exchange resin to thereby allow the basic anion exchange rein to adsorb shikimic acid, and eluting the shikimic acid with an aqueous basic solution.

Such basic anion exchange resins for use in the present invention include, but are not limited to, "Diaion" WK10, "Diaion" WK11, "Diaion" WK20, "Diaion" WA10, "Diaion" WA11, "Diaion" WA20, "Diaion" WA21, and "Diaion" WA30 (trade names of Mitsubishi Chemical Corporation), "Amberlite" IR-4B, and "Amberlite" IR-45 (trade names of Rohm and Haas Co.), "DOW-X" 3, "DOW-X" MWA-1, and "DOW-X" WGRA (trade names of The Dow Chemical Co.).

Preferred eluents for eluting shikimic acid with an aqueous basic solution after the adsorption of shikimic acid by the basic anion exchange resin include aqueous solutions of metal hydroxides such as sodium hydroxide, potassium hydroxide, calcium hydroxide, magnesium hydroxide, aluminium hydroxide, and barium hydroxide, of which an aqueous sodium hydroxide solution is more preferred. The concentration of the eluent aqueous basic solution is preferably 0.0001 N to 5 N, and more preferably 0.01 N to 2 N. The pH of the eluate is preferably 8 or more, and more preferably 10 or more.

The eluate may be concentrated or be left as intact without concentration, as far as its pH can be maintained at 8 or more, but should be more preferably concentrated.

In the present invention, the total amount of the solution containing shikimic acid to brought into contact with the basic anion exchange resin should preferably be such that adsorbed components in the solution does not exceed the exchange capacity of the resin. Preferably, the solution containing shikimic acid is brought into contact with the basic anion exchange resin and water is then passed through the resin in an amount about two times the total capacity of the basic anion exchange resin to remove non-adsorbed components, and an aqueous basic solution such as a 0.1 N aqueous sodium hydroxide solution is passed through the resin in an amount two to three times the total capacity of he basic anion exchange resin to thereby recover the eluate.

When the solution containing shikimic acid is subjected to a basic condition as described above, an impurity 3-dehydroshikimic acid is dehydrated and is isomerized into, for example, protocatechuic acid. Shikimic acid has significantly different solubility and other physical properties from protocatechuic acid and other impurities and can easily be separated from protocatechuic acid and other impurities by the use of, for example, crystallization, extraction with an organic solvent, activated carbon, chelate resins, synthetic adsorbents, and resins for chromatographic separation.

Accordingly, the invented process can produce a higher purity shikimic acid in a higher yield than conventional techniques, by isomerizing 3-dehydroshikimic acid into easily-separable impurities such as protocatechuic acid, which 3-dehydroshikimic acid contaminates shikimic acid and cannot be significantly separated.

The present invention will now be illustrated in further detail with reference to several examples below.

### EXAMPLE 1

### (Isolation of Shikimic Acid-secreting Mutant)

Cells of *Citrobacter freundii* OFPR36-158 were treated with 300 µg/ml N-methyl-N'-nitro-N-nitrosoguanidine at 30°C for 10 minutes according to a conventional procedure, were appropriately diluted, and were plated on a medium shown in Table 1, and the plated cells were cultured at 30°C for two days. Next, colonies of mutagen-treated *Citrobacter freundii* OFPR36-158 formed on the medium shown in Table 1 were replicated on media (plate media) shown in Tables 2 and 3. The replicated colonies were cultured at 30°C for three days. No colony grew on the medium shown in Table 2. A colony (4AA-12 strain (FERM BP-6722)) grew on the medium shown in Table 3 was isolated as a candidate strain for the production of shikimic acid.

**Table 1**

| | |
|---|---|
| Bacto-triptone | 10 g/L |
| Bacto-yeast extract | 5 g/L |
| Sodium chloride | 5 g/L |
| Agar | 15 g/L |
| pH 7.5 (adjusted with NaOH) | |

**Table 2**

| | |
|---|---|
| Glucose | 10 g/L |
| Monopotassium phosphate | 3 g/L |
| Dipotassium phosphate | 7 g/L |
| Ammonium sulfate | 1 g/L |
| Magnesium sulfate heptahydrate | 0.1 g/L |
| Agar | 15 g/L |

**Table 3**

| | |
|---|---|
| Glucose | 10 g/L |
| Monopotassium phosphate | 3 g/L |
| Dipotassium phosphate | 7 g/L |
| Ammonium sulfate | 1 g/L |
| Magnesium sulfate heptahydrate | 0.1 g/L |
| L-Tyrosine | 0.05 g/L |
| L-Phenylalanine | 0.05 g/L |
| L-Tryptophan | 0.05 g/L |
| p-Aminobenzoic acid | 1 mg/L |
| Agar | 15 g/L |

### EXAMPLE 2

### (Culturing of Shikimic Acid-secreting Mutant and Production of Shikimic Acid by Fermentation)

Each of strains shown in Table 5 was precultured in a medium shown in Table 1 (a liquid medium having a composition shown in Table 1 except for agar) at 30°C for 24 hours with shaking, and the resulting preculture was inoculated into 50 ml of a medium having a composition shown in Table 4 in a 500-ml Erlenmeyer flask, which medium had been steam-sterilized at 115°C for 10 minutes. The cells were cultured for 144 hours with shaking with 30 cm reciprocating span at 180 rpm.

After the completion of culturing, the cultured cells and calcium carbonate were eliminated, and a shikimic acid concentration of the filtrate was determined by HPLC technique (column: Shimadzu SCR-101H, mobile phase: 0.1 M phosphoric acid at 1 mL/min., detection: 254 nm). The results are shown in Table 5.

**Table 4**

| | |
|---|---|
| Glucose | 50 g/L |
| Monopotassium phosphate | 1 g/L |
| Ammonium sulfate | 25 g/L |
| Magnesium sulfate heptahydrate | 0.4 g/L |
| L-Tyrosine | 0.1 g/L |
| L-Phenylalanine | 0.1 g/L |
| L-Tryptophan | 0.1 g/L |
| p-Aminobenzoic acid | 1 mg/L |
| Ferrous sulfate heptahydrate | 9.9 mg/L |
| Manganese sulfate tetrahydrate | 7.2 mg/L |
| Zinc chloride | 25 mg/L |
| Copper sulfate heptahydrate | 0.5 mg/L |
| Calcium carbonate | 20 g/L |

**Table 5**

| Strains | Accumulated shikimic acid concentration (g/L) |
|---|---|
| FPR36-158 | No detection |
| 4AA-12(FERM BP-6722) | 4.1 |

### EXAMPLE 3

### (Isolation of Pyruvic Acid Analogue-resistant Mutant)

Cells of *Citrobacter freundii* 4AA-12 (FERM BP-6722) were treated with 300 µg/ml N-methyl-N'-nitro-N-nitrosoguanidine at 30°C for 10 minutes according to a conventional procedure, were appropriately diluted, and were plated on a plate medium having a composition shown in Table 2 and further comprising 2.5 mM bromopyruvic acid. The plated cells were cultured at 30°C for four days.

Next, colonies of mutants of mutagen-treated *Citrobacter freundii* 4AA-12 formed on the medium were purified on a medium shown in Table 2, and a resulting colony formed thereon was isolated as a candidate strain for the production of shikimic acid.

### EXAMPLE 4

### (Culturing of Pyruvic Acid Analogue-resistant Mutant and Production of Shikimic Acid by Fermentation)

Each of strains shown in Table 6 was precultured in a medium shown in Table 1 (a liquid medium having a composition shown in Table 2 except for agar) at 30°C for 24 hours with shaking, and the resulting preculture was inoculated into 50 ml of a medium having a composition shown in Table 4 in a 500-ml Erlenmeyer flask, which medium had been steam-sterilized at 115°C for 10 minutes. The cells were cultured for 144 hours with shaking with 30 cm reciprocating span at 180 rpm.

After the completion of culturing, the cultured cells and calcium carbonate were eliminated, and a shikimic acid concentration of the filtrate was determined by HPLC technique (column: Shimadzu SCR-101H, mobile phase: 0.1 M phosphoric acid at 1 mL/min., detection: 254 nm). The results are shown in Table 6.

**Table 6**

| Strains | Accumulated shikimic acid concentration (g/L) |
|---|---|
| 4AA-12(FERM BP-6722) | 4.5 |
| BPR-3(FERM BP-6723) | 9.3 |

### EXAMPLE 5

### (Resistant Level of Pyruvic Acid Analogue-resistant Mutant to Pyruvic Acid Analogue)

Bromopyruvic acid was added to the medium shown in Table 3 in concentrations indicated in Table 7 to prepare media. Each of strains shown in Table 7 was plated respectively on the media and was cultured at 30°C for four days. The results are shown in Table 7, indicating that the pyruvic acid analogue-resistant mutant BPR-3 acquired resistance to bromopyruvic acid.

**Table 7**

| Strains | Growth | | | |
|---|---|---|---|---|
| | Bromopyruvic acid concentration (mM) | | | |
| | 0 | 1 | 2.5 | 5 |
| 4AA-12(FERM BP-6722) | ○ | X | X | X |
| BPR-3(FERM BP-6723) | ○ | ○ | ○ | △ |

| | | | | |
|---|---|---|---|---|
| X: Not grown | | | | |
| △: Slightly grown | | | | |
| ○ : Grown | | | | |

### EXAMPLE 6

### (Isolation of Succinic Acid- or Glutamic Acid-requiring Mutant)

Cells of *Citrobacter freundii* 4AA-12 (FERM BP-6722) were treated with 300 µg/ml N-methyl-N'-nitro-N-nitrosoguanidine at 30°C for 10 minutes according to a conventional procedure, were appropriately diluted, and were plated on a plate medium having a composition shown in Table 1. The plated cells were cultured at 30°C for two days.

Colonies of mutants of mutagen-treated *Citrobacter freundii* 4AA-12 formed on the medium shown in Table 1 were replicated on plate media including a medium shown in Table 3 and two media, i.e., media having the composition shown in Table 3 and further containing 5 g/L of sodium succinate trihydrate or 1 g/L of L-glutamic acid, and were cultured at 30°C for two days. A colony that grew on a medium containing succinic acid or glutamic acid but did not grow on the medium containing neither succinic acid nor glutamic acid was isolated as a candidate for a succinic acid- or glutamic acid-requiring mutant.

### EXAMPLE 7

### (Certification on Succinic Acid- or Glutamic Acid-requiring Mutant)

The succinic acid- or glutamic acid-requiring mutant candidate isolated in Example 6 was slightly plated respectively on a medium shown in Table 3, an agar medium having a composition shown in Table 3 and further containing 5 g/L of sodium succinate trihydrate, an agar medium having a composition shown in Table 3 and further containing 1 g/L of sodium L-glutamate, and an agar medium having a composition shown in Table 3 and further containing both substances, and the plated cells were cultured at 30°C for four days. The results are shown in Table 8, indicating that the mutant SUC-87 did not grow on a medium containing neither succinic acid nor glutamic acid but grew on media containing either of these substances and that this mutant was a nutrient requiring (auxotrophic) mutant.

**Table 8**

| Strains | Additives | | | |
|---|---|---|---|---|
| | None | Succinic acid | L-Glutamic acid | Succinic acid L-glutamic acid |
| 4AA-12 (FERM BP-6722) | ○ | ○ | ○ | ○ |
| SUC-87 (FERM BP-6724) | X | ○ | ○ | ○ |

| | | | | |
|---|---|---|---|---|
| X: Not grown | | | | |
| △: Slightly grown | | | | |
| ○: Grown | | | | |

### EXAMPLE 8

### (Culturing of Succinic Acid- or Glutamic Acid-requiring Mutant and Production of Shikimic Acid by Fermentation)

Each of strains shown in Table 9 was precultured in a medium shown in Table 1 (a liquid medium having a composition shown in Table 1 except for agar) at 30°C for 24 hours with shaking, and the resulting preculture was inoculated into 50 ml of a medium having a composition shown in Table 4 in a 500-ml Erlenmeyer flask, which medium had been steam-sterilized at 115°C for 10 minutes. The cells were cultured for 144 hours with shaking with 30 cm reciprocating span at 180 rpm.

After the completion of culturing, the cultured cells and calcium carbonate were eliminated, and a shikimic acid concentration of the filtrate was determined by HPLC technique (column: Shimadzu SCR-101H, mobile phase: 0.1 M phosphoric acid at 1 mL/min., detection: 254 nm). The results are shown in Table 9.

**Table 9**

| Strains | Accumulated shikimic acid concentration (g/L) |
|---|---|
| 4AA-12(FERM BP-6722) | 4.5 |
| SUC-87(FERM BP-6724) | 8.8 |

### EXAMPLE 9

### (Isolation of Vitamin B1 Analogue-resistant Mutant)

Cells of *Citrobacter freundii* 4AA-12 (FERM BP-6722) were treated with 300 µg/ml N-methyl-N'-nitro-N-nitrosoguanidine at 30°C for 10 minutes according to a conventional procedure, were appropriately diluted, and were plated on a plate medium having a composition shown in Table 3 and further containing 10 mM oxythiamine. The plated cells were cultured at 30°C for three days.

Next, colonies of mutants of mutagen-treated *Citrobacter freundii* 4AA-12 formed on the medium were purified on a medium shown in Table 3, and a resulting colony formed thereon was isolated as a candidate strain for the production of shikimic acid.

### EXAMPLE 10

### (Culturing of Vitamin B1 Analogue-resistant Mutant and Production of Shikimic Acid by Fermentation)

Each of strains shown in Table 11 was precultured in a medium shown in Table 1 (a liquid medium having a composition shown in Table 1 except for agar) at 30°C for 24 hours with shaking, and the resulting preculture was inoculated into 50 ml of a medium having a composition shown in Table 4 in a 500 ml Erlenmeyer flask, which medium had been steam-sterilized at 115°C for 10 minutes. The cells were cultured for 100 hours with shaking with 30 cm reciprocating span at 180 rpm.

After the completion of culturing, the cultured cells and calcium carbonate were eliminated, and a shikimic acid concentration of the filtrate was determined in the same manner as In Example 2. The results are shown in Table 10.

**Table 10**

| Strains | Accumulated shikimic acid concentration (g/L) |
|---|---|
| 4AA-12(FERM BP-6722) | 4.5 |
| OT -H24(FERM BP-6711) | 9.3 |

### EXAMPLE 11

### (Resistant Level of Vitamin B1 Analogue-resistant Mutant to Vitamin B1 Analogue)

Each of strains shown in Table 11 was plated on a medium shown in Table 3 and the plated cells were cultured at 30°C for three days. The results are shown in Table 11, indicating that the vitamin B1 analogue-resistant mutant OT-H24 acquired resistance to a vitamin B1 analogue oxythiamine.

**Table 11**

| Strains | Growth | | | |
|---|---|---|---|---|
| | Oxythiamine concentration (mM) | | | |
| | 0 | 5 | 10 | 15 |
| 4AA-12(FERM BP-6722) | ○ | X | X | X |
| OT-H24(FERM BP-6723) | ○ | ○ | ○ | △ |

| | | | | |
|---|---|---|---|---|
| X: Not grown | | | | |
| △: Slightly grown | | | | |
| ○: Grown | | | | |

### EXAMPLE 12

### (Isolation of Mutant Resistant to Highly Concentrated Shikimic Acid)

Cells of *Citrobacter freundii* 4AA-12 (FERM BP-6722) were treated with 300 µg/ml N-methyl-N'-nitro-N-nitrosoguanidine at 30°C for 10 minutes according to a conventional procedure, were appropriately diluted, and were plated on a plate medium having a composition shown in Table 3 and further containing 35 g/l shikimic acid. The plated cells were cultured at 30°C for three days.

Next, colonies of mutagen-treated *Citrobacter freundii* HSK-10 (FERM BP-6712) mutant formed on the medium were purified on a medium shown in Table 3, and a resulting colony formed thereon was isolated as a candidate strain for the production of shikimic acid.

### EXAMPLE 13

### (Culturing of Mutant Resistant to Highly Concentrated Shikimic Acid and Production of Shikimic Acid by Fermentation)

Each of strains shown in Table 12 was precultured in a liquid medium having a composition shown in Table 1 except for agar at 30°C for 24 hours with shaking, and the resulting preculture was inoculated into 50 ml of a medium having a composition shown in Table 4 in a 500-ml Erlenmeyer flask, which medium had been steam-sterilized at 115°C for 10 minutes. The cells were cultured for 100 hours with shaking with 30 cm reciprocating span at 180 rpm.

After the completion of culturing, the cultured cells and calcium carbonate were eliminated, and a shikimic acid concentration of the filtrate was determined in the same manner as in Example 2. The results are shown in Table 12.

**Table 12**

| Strains | Accumulated shikimic acid concentration (g/L) |
|---|---|
| 4AA-12(FERM BP-6722) | 4.5 |
| HSK-10 (FERM BP-6712) | 10.0 |

### EXAMPLE 14

### (Resistant Level of Highly Concentrated Shikimic Acid-resistant Mutant)

Each of strains shown in Table 13 was plated on media each having a composition shown in Table 3 and further containing shikimic acid in a concentration indicated in Table 13. The plated cells were cultured at 30°C for three days. The results are shown in Table 13, indicating that the highly concentrated shikimic acid-resistant mutant HSK-10 acquired resistance to a highly concentrated shikimic acid.

**Table 13**

| Strains | Growth | | | |
|---|---|---|---|---|
| | Shikimic acid concentration (g/l) | | | |
| | 0 | 15 | 35 | 50 |
| 4AA-12(FERM BP-6722) | ○ | △ | X | X |
| HSK-10(FERM BP-6712) | ○ | ○ | ○ | △ |

| | | | | |
|---|---|---|---|---|
| X: Not grown | | | | |
| △: Slightly grown | | | | |
| ○: Grown | | | | |

### EXAMPLE 15

### (Culturing of Shikimic Acid-secreting Mutant and Production of Shikimic Acid by Fermentation in the Presence of Transition Metal)

Cells of *Citrobacter freundii* 4AA-12 (FERM BP-6722) strain were precultured in a medium shown in Table 1 at 30°C for 48 hours with shaking, and the resulting preculture was inoculated into 50 ml of each of media having a composition shown in Table 4 and further containing a compound shown in Table 14 in a 500 ml Erlenmeyer flask, which media had been steam-sterilized at 121°C for 20 minutes. The cells were cultured for 72 hours with shaking with 30 cm reciprocating span at 180 rpm.

After the completion of culturing, the cultured cells and calcium carbonate were eliminated, and a shikimic acid concentration of the filtrate was determined by HPLC technique (column: Shimadzu SCR-101H, mobile phase: 0.1 M phosphoric acid at 1 mL/min., detection: 254 nm). The results are shown in Table 14.

**Table 14**

| | Accumulated shikimic acid concentration (g/L) |
|---|---|
| No zinc chloride added | 1.7 |
| 2.5 mg/L Zinc chloride added | 4.5 |
| 25 mg/L | 3.8 |
| 100 mg/L | 5.2 |
| 500 mg/L | 4.7 |

### EXAMPLE 16

A supernatant of one liter of the culture obtained in Example 15 was passed through a cation exchange resin Lewatit S100 (produced by Rohm and Haas Co.) and flow-through fractions thereof were pooled. The pooled fractions were then passed through an anion exchange resin Diaion WA30 (produced by Mitsubishi Chemical Corporation), and was desorbed with 0.1 N NaOH, and fractions containing shikimic acid were pooled. The pooled fractions were again passed through Lewatit S100 and flow-through fractions thereof were concentrated to 100 mL. Shikimic acid in the concentrated solution was extracted with three portions of 100 mL of n-heptanol containing 0.3 M tridecylamine, and oleic acid was then added to the extract to a concentration of 0.15 M and the resulting mixture was back-extracted into aqueous phase. The resulting aqueous solution was treated with an activated carbon, was crystallized by concentration, and was recrystallized in isopropanol to yield 2.3 g of crystalline shikimic acid having a purity of 97% or more.

### EXAMPLE 17

### (Production of Shikimic Acid by Purification Using Basic Anion Exchange Resin)

Cells of *Citrobacter freundii* 4AA-12 (FERM BP-6722) were precultured in a medium shown in Table 1 at 30°C for 48 hours with shaking, and the resulting preculture was inoculated into 50 ml of a medium having a composition shown in Table 4 in a 500-ml Erlenmeyer flask, which media had been steam-sterilized at 121°C for 20 minutes. The cells were cultured for 72 hours with shaking with 30 cm reciprocating span at 180 rpm.

A supernatant of one liter of the culture obtained above was passed through 180 ml of a cation exchange resin Lewatit S100 (produced by Rohm and Haas Co.) and flow-through fractions thereof were pooled. The pooled fractions were further passed through 180 ml of an anion exchange resin Diaion WA30 (produced by Mitsubishi Chemical Corporation), and was eluted with 0.2 N H₂SO₄ and 0.1 N NaOH respectively, and fractions containing shikimic acid were pooled. The pooled fractions were again passed through 180 ml of Lewatit S100, and flow-through fractions thereof were concentrated to 100 mL. A shikimic acid concentration was determined by HPLC technique (column: Shimadzu SCR-101H, mobile phase: 0.1 M phosphoric acid at 1 mL/min., detection: 254 nm). The results are shown in Table 15.

**Table 15**

| Peak Intensity Ratio on HPLC (3-dehydroshikimic acid/shikimic acid) | |
|---|---|
| Before anion exchange | 4.57 |
| 0.2 N Sulfuric acid | 7.15 |
| 0.1 N Sodium hydroxide | 0.005 |

Shikimic acid in the resulting eluate was extracted with three portions of 100 mL of n-heptanol containing 0.3 M tridecylamine, and oleic acid was then added to the extract to a concentration of 0.15 M and the resulting mixture was back-extracted into aqueous phase. The resulting aqueous solution was treated with an activated carbon, was crystallized by concentration, and was recrystallized in isopropanol to yield 2.3 g of crystalline shikimic acid having a purity of 97% or more.

### EXAMPLE 18

A supernatant of one liter of the culture obtained in Example 2 using the strain 4AA-12 was passed through 180 ml of a cation exchange resin Lewatit S100 (produced by Rohm and Haas Co.) and flow-through fractions thereof were pooled. The pooled fractions were further passed through 180 ml of an anion exchange resin Diaion WA30 (produced by Mitsubishi Chemical Corporation), and was eluted with 0.1 N NaOH, and fractions containing shikimic acid were pooled. The pooled fractions were stirred at room temperature (25°C to 30°C) only between hour zero to hour four and between hour 17 to hour 24. The pH and concentrations of individual components are shown in the table. As there is no authentic sample of 3-dehydroshikimic acid, the concentration of 3-dehydroshikimic acid is indicated in area ratio (%) to shikimic acid as determined by HPLC technique (column: Shimadzu SCR-101H, mobile phase: 0.1 v/v % phosphoric acid at 1 mL/min., detection: 210 nm).

**Table 16**

| Time (hr) | pH | Shikimic acid (g/L) | 3-Dehydroshikimic acid (%) | Protocatechuic acid (g/L) |
|---|---|---|---|---|
| 0 | 10.12 | 10.40 | 1.67 | 0.016 |
| 1 | 9.86 | 10.18 | 1.64 | 0.018 |
| 3 | 9.61 | 10.42 | 1.30 | 0.015 |
| 17 | 9.15 | 10.35 | 1.07 | 0.019 |
| 24 | 8.77 | 10.41 | 0.97 | 0.012 |

### EXAMPLE 19

The same solution as in Example 18 was stirred at 50°C. The pH and concentrations (g/L) of individual components are shown in the table. The concentration of 3-dehydroshikimic acid is indicated in area ratio to shikimic acid determined by HPLC technique.

**Table 17**

| Time (hr) | pH | Shikimic acid (g/L) | 3-Dehydroshikimic acid (%) | Protocatechuic acid (g/L) |
|---|---|---|---|---|
| 0 | 10.01 | 10.41 | 1.38 | 0.031 |
| 1 | 9.37 | 10.28 | 0.97 | 0.042 |
| 2 | 8.99 | 10.35 | 0.82 | 0.044 |
| 5 | 8.30 | 10.37 | 0.74 | 0.037 |

### EXAMPLE 20

The same solution as in Example 18 was left at 80°C without stirring. The pH and concentrations (g/L) of individual components are shown in the table. The concentration of 3-dehydroshikimic acid is indicated in area ratio to shikimic acid determined by HPLC technique.

**Table 18**

| Time (hr) | pH | Shikimic acid (g/L) | 3-Dehydroshikimic acid (%) | Protocatechuic acid (g/L) |
|---|---|---|---|---|
| 0 | 10.01 | 10.41 | 1.38 | 0.031 |
| 1 | 9.37 | 10.39 | 0.27 | 0.26 |
| 2 | 8.99 | 10.23 | 0.18 | 0.28 |
| 5 | 8.30 | 10.41 | 0.07 | 0.30 |

### EXAMPLE 21

The same solution as in Example 18 was concentrated for three hours with a rotatory evaporator on a bath at 60°C to about one tenth. The pH and concentrations (g/L) of individual components are shown in Table 19. The concentration of 3-dehydroshikimic acid is indicated in area ratio to shikimic acid determined by HPLC technique.

**Table 19**

| | pH | Shikimic acid (g/L) | 3-Dehydroshikimic acid (%) | Protocatechuic acid (g/L) |
|---|---|---|---|---|
| Before concentration | 10.12 | 10.40 | 1.67 | 0.016 |
| After concentration | 9.51 | 100.80 | 0.43 | 1.64 |

### Industrial Applicability

The invented process can more economically produce shikimic acid than conventional processes.

Shikimic acid is used in, for example, pharmaceutical materials.

## Claims

1. A microorganism belonging to the genus Citrobacter and capable of extracellularly secreting shikimic acid.

2. A microorganism belonging to the genus Citrobacter according to claim 1, wherein said microorganism belonging to the genus Citrobacter is *Citrobacter freundii*.

3. A microorganism according to claim 1, having any of the following properties:
(1) the property of requiring an aromatic amino acid and/or p-aminobenzoic acid for its growth;
(2) the property of being resistant to a pyruvic acid analogue;
(3) the property of requiring succinic acid for its growth;
(4) the property of requiring glutamic acid for its growth;
(5) the property of being resistant to a vitamin B1 analogue; and
(6) the property of being resistant to a highly concentrated shikimic acid.

4. A microorganism according to claim 3, wherein said pyruvic acid analogue is bromopyruvic acid.

5. A microorganism according to claim 3, wherein said vitamin B1 analogue is oxythiamine.

6. A process for producing shikimic acid, using the microorganism according to one of claims 1 to 5.

7. A process for producing shikimic acid, comprising the steps of culturing the microorganism according to one of claims 1 to 5 in a culture medium in the presence of a transition metal to thereby accumulate the shikimic acid in the culture medium, and isolating and recovering the shikimic acid from the culture medium.

8. A process for producing shikimic acid according to claim 7, wherein said transition metal is at least one selected from metals of Group VIIB, Group VIII, Group IB, and Group IIB.

9. A process for producing shikimic acid according to claim 8, wherein said transition metal is zinc.

10. A process for producing shikimic acid according to claim 9, wherein the concentration of zinc is in a range from 0.1 mg/L to 500 mg/L.

11. A process for producing shikimic acid, comprising the step of subjecting a solution containing shikimic acid to a basic condition to thereby purify shikimic acid.

12. A process for producing shikimic acid, comprising the step of subjecting a solution containing shikimic acid to a basic condition to thereby purify shikimic acid, said shikimic acid in said solution being produced by a process for producing shikimic acid using the microorganism according to one of claims 1 to 5.

13. A process for producing shikimic acid according to claim 11 or 12, wherein the pH in said basic condition is 8 or more.

14. A process for producing shikimic acid according to claim 11 or 12, wherein the solution containing shikimic acid is brought into contact with an anion exchange resin so as to allow the anion exchange resin to adsorb shikimic acid, and eluting shikimic acid with an aqueous basic solution, to thereby subject the solution containing shikimic acid to a basic condition.
